# EUROPEAN PATENT APPLICATION

(11) **EP 4 772 533 A1**
(43) Date of publication of application: **08.07.2026**
(21) Application number: 24857678.7
(22) Date of filing: 01.03.2024
(51) Int. Cl.: C07K 14/78, C12N 15/12, A61K 8/65

(54) **RECOMBINANT HUMANIZED TYPE III TRIPLE HELICAL COLLAGEN, PREPARATION METHOD THEREFOR, AND USE THEREOF**

(30) Priority: 28.08.2023 CN 202311094520
(71) Applicant: Kexing Medical Device Co., Ltd., Guangdong 518000 (CN)
(72) Inventor: QIN, Suofu, Jinan, Shandong 250200 (CN); XU, Chen, Jinan, Shandong 250200 (CN); DENG, Feiyan, Jinan, Shandong 250200 (CN); LU, Haifeng, Jinan, Shandong 250200 (CN); SHANG, Wei, Jinan, Shandong 250200 (CN); YIN, Jiangfeng, Jinan, Shandong 250200 (CN); HUANG, Huiyu, Jinan, Shandong 250200 (CN); ZHOU, Xinrong, Jinan, Shandong 250200 (CN)
(74) Representative: Herrero & Asociados, S.L.
(86) International application number: PCT/CN2024/079679
(87) International publication number: WO 2025/044123

(57) **Abstract**

The present application proposes the use and preparation method of recombinant humanized type III triple-helical collagen. Specifically, the present application proposes a recombinant collagen monomer. According to embodiments of the present application, the recombinant collagen monomer includes at least one collagen active fragment, and the collagen active fragment includes at least three GER sites and/or GEK sites and/or RGD sites and/or GEN. These three adhesion sites are the same or different, and the collagen active fragment is derived from amino acids at positions 168 to 1196 of a collagen.

## Description

### Priority Information

The present application claims priority to and the benefits of Chinese Patent Application No. 2023110945209, filed with the China National Intellectual Property Administration on August 28, 2023, the entire content of which is incorporated herein by reference.

### Technical Field

The present application relates to the field of biotechnology, and specifically to a recombinant humanized type III triple-helical collagen and a preparation method and use thereof.

### Background

Natural collagen is the most abundant class of high-molecular-weight proteins in organisms, accounting for approximately 20-30% of total protein. To date, approximately 28 types of natural collagen have been discovered, which are widely distributed in the connective tissues of skin, bone, tendons, and various organs, and participate in various vital activities such as wound repair, skin aging, blood coagulation, and joint lubrication. As an important biomaterial, collagen has extensive application value in the fields of medicine, aesthetics, the food industry, and the like.

Natural type I and type III collagens are fibrous collagens with complex multi-level structures. They have a large molecular weight, and the amino acid sequence of the helical domain in the molecule is a Gly-X-Y repeat sequence, where Y is usually hydroxyproline. Three homologous or heterologous protein molecules are arranged into a triple-helical structure through intermolecular forces such as hydrogen bonds and electrostatic forces, and further stacked and arranged into higher-order fibrous structures.

At present, the sources of collagen on the market are mainly animals such as pigs, cattle, and fish. Animal-derived collagen is mainly processed and extracted through acid hydrolysis, alkaline hydrolysis, and enzymatic hydrolysis processes. However, animal-derived collagen has the following disadvantages: 1) since there are differences in amino acid sequences between animal-derived collagen and human collagen, immunogenicity exists, and thus there is a risk of allergic reactions; 2) animal-derived collagen may contain viruses, such as bovine spongiform encephalopathy (BSE) prions and the like; 3) during the extraction process, the collagen structure is damaged to a certain extent, which leads to the weakening of the original biological activity; 4) religious and ethical issues exist.

Therefore, there is an urgent need to develop a new method for preparing collagen to overcome the above-mentioned problems.

### Summary of the Invention

The present application aims to address at least one of the technical problems existing in the prior art, at least to a certain extent.

In recent years, with the development of synthetic biology, researchers have utilized gene recombination expression technology to mass-produce recombinant human collagen using microorganisms (such as Escherichia coli and yeast) as cell factories. Compared with traditional animal collagen extraction processes, recombinant collagen production has the following advantages: 1) the amino acid sequence of recombinant human collagen is identical to that of human natural collagen, thereby eliminating safety hazards of animal immunogenicity; 2) transmission of animal-derived viruses is avoided; 3) the microbial fermentation process has low cost and a short production cycle, and is suitable for large-scale production. In recent years, the Pichia pastoris expression system has been widely used in the production of recombinant collagen. The inventor(s) have found that the Pichia pastoris expression system possesses the advantages of both prokaryotic and eukaryotic expression systems, such as easy operation, high expression level, and low cost, which are conducive to large-scale industrial production, while providing a certain degree of post-translational modification for exogenous proteins. Through genetic engineering means, the inventor(s) designed and modified the Pichia pastoris expression system and found that the humanized collagen expressed by yeast has the ability to form a triple-helical conformation. Humanized collagen with a triple-helical conformation and biological activity is an advanced biomaterial close to natural collagen. In addition, the inventor(s) also found that the protein secretion efficiency of Pichia pastoris is relatively high, while its own protein secretion is relatively low; therefore, high-level secretion of exogenous proteins can be achieved, which are easy to purify, and toxic side effects caused by the accumulation of fermentation products can be avoided.

Therefore, in a first aspect of the present application, the present application proposes a recombinant collagen monomer. According to embodiments of the present application, the recombinant collagen monomer includes at least one collagen active fragment, wherein the collagen active fragment includes at least three GER sites and/or GEK sites and/or RGD sites and/or GEN sites. Through continuous experiments, the inventor(s) have found that peptide segments in natural collagen including at least three adhesion-related sites selected from GER sites and/or GEK sites and/or RGD sites and/or GEN sites are collagen adhesion active sites. For peptide segments obtained by artificially breaking natural collagen, only peptide segments including at least three adhesion-related sites selected from GER sites and/or GEK sites and/or RGD sites and/or GEN sites possess adhesion activity or expression or secretion activity in recombinant cells.

According to embodiments of the present application, the recombinant collagen monomer further includes at least one of the following additional technical features.

According to embodiments of the present application, the collagen active fragment is derived from amino acids at positions 168 to 1196 of a collagen.

According to embodiments of the present application, the collagen active fragment includes three GER sites and/or GEK sites and/or RGD sites and/or GEN sites.

According to embodiments of the present application, the three GER sites and/or GEK sites and/or RGD sites and/or GEN sites are the same or different. For example, three GER sites, or three GEK sites, or two GEK sites and one GER site, etc., may be included.

According to embodiments of the present application, the collagen active fragment is a peptide segment between amino acid position 1089 and amino acid position 1172 of a collagen. The inventor(s) have found that within this range, the adhesion activity of the collagen active fragment is highest, and the expression and secretion are highest, which lays a foundation for subsequent preparation of the recombinant collagen with high cell adhesion activity.

According to embodiments of the present application, the collagen active fragment is a peptide segment between amino acid position 234 and amino acid position 296 of a collagen.

According to embodiments of the present application, the collagen active fragment is a peptide segment between amino acid position 273 and amino acid position 311 of a collagen.

According to embodiments of the present application, the collagen active fragment is a peptide segment between amino acid position 288 and amino acid position 452 of a collagen.

According to embodiments of the present application, the collagen active fragment is a peptide segment between amino acid position 300 and amino acid position 485 of a collagen.

According to embodiments of the present application, the collagen active fragment is a peptide segment between amino acid position 444 and amino acid position 503 of a collagen.

According to embodiments of the present application, the collagen active fragment is a peptide segment between amino acid position 483 and amino acid position 512 of a collagen.

According to embodiments of the present application, the collagen active fragment is a peptide segment between amino acid position 501 and amino acid position 599 of a collagen.

According to embodiments of the present application, the collagen active fragment is a peptide segment between amino acid position 507 and amino acid position 686 of a collagen.

According to embodiments of the present application, the collagen active fragment is a peptide segment between amino acid position 591 and amino acid position 731 of a collagen.

According to embodiments of the present application, the collagen active fragment is a peptide segment between amino acid position 678 and amino acid position 806 of a collagen.

According to embodiments of the present application, the collagen active fragment is a peptide segment between amino acid position 726 and amino acid position 830 of a collagen.

According to embodiments of the present application, the collagen active fragment is a peptide segment between amino acid position 795 and amino acid position 833 of a collagen.

According to embodiments of the present application, the collagen active fragment is a peptide segment between amino acid position 798 and amino acid position 839 of a collagen.

According to embodiments of the present application, the collagen active fragment is a peptide segment between amino acid position 852 and amino acid position 866 of a collagen.

According to embodiments of the present application, the collagen active fragment is a peptide segment between amino acid position 828 and amino acid position 932 of a collagen.

According to embodiments of the present application, the collagen active fragment is a peptide segment between amino acid position 861 and amino acid position 995 of a collagen.

According to embodiments of the present application, the collagen active fragment is a peptide segment between amino acid position 927 and amino acid position 1094 of a collagen.

According to embodiments of the present application, the collagen active fragment is a peptide segment between amino acid position 987 and amino acid position 1103 of a collagen.

According to embodiments of the present application, the collagen active fragment is a peptide segment between amino acid position 1089 and amino acid position 1172 of a collagen, and/or a peptide segment between amino acid position 234 and amino acid position 296 of the collagen, and/or a peptide segment between amino acid position 273 and amino acid position 311 of the collagen, and/or a peptide segment between amino acid position 288 and amino acid position 452 of the collagen, and/or a peptide segment between amino acid position 300 and amino acid position 485 of the collagen, and/or a peptide segment between amino acid position 444 and amino acid position 503 of the collagen, and/or a peptide segment between amino acid position 483 and amino acid position 512 of the collagen, and/or a peptide segment between amino acid position 501 and amino acid position 599 of the collagen, and/or a peptide segment between amino acid position 507 and amino acid position 686 of the collagen, and/or a peptide segment between amino acid position 591 and amino acid position 731 of the collagen, and/or a peptide segment between amino acid position 678 and amino acid position 806 of the collagen, and/or a peptide segment between amino acid position 726 and amino acid position 830 of the collagen, and/or a peptide segment between amino acid position 795 and amino acid position 833 of the collagen, and/or a peptide segment between amino acid position 798 and amino acid position 839 of the collagen, and/or a peptide segment between amino acid position 852 and amino acid position 866 of the collagen, and/or a peptide segment between amino acid position 828 and amino acid position 932 of the collagen, and/or a peptide segment between amino acid position 861 and amino acid position 995 of the collagen, and/or a peptide segment between amino acid position 927 and amino acid position 1094 of the collagen, and/or a peptide segment between amino acid position 987 and amino acid position 1103 of the collagen.

According to embodiments of the present application, the collagen active fragment has an amino acid sequence as shown in SEQ ID NO: 2, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, or SEQ ID NO: 24.

According to embodiments of the present application, the recombinant collagen monomer includes 2 to 12 of the collagen active fragments.

According to embodiments of the present application, the recombinant collagen monomer includes six of the collagen active fragments.

According to embodiments of the present application, the collagen active fragments are the same or different.

According to embodiments of the present application, the 2 to 12 collagen active fragments are connected directly or indirectly.

According to embodiments of the present application, it further includes a stabilization sequence, wherein the stabilization sequence is connected to a C-terminus of the at least one collagen active fragment, thereby increasing the stability of the recombinant collagen monomer.

According to embodiments of the present application, an amino acid sequence of the stabilization sequence is SEQ ID NO: 6.

According to embodiments of the present application, the recombinant collagen monomer has an amino acid sequence as shown in SEQ ID NO: 3 or SEQ ID NO: 25.

In a second aspect of the present application, the present application proposes a recombinant collagen. According to embodiments of the present application, the recombinant collagen includes three of the recombinant collagen monomers according to the first aspect. Therefore, the recombinant collagen has high cell adhesion activity and good hydrophilicity, and can be widely applied in various aspects such as beauty, medicine, and tissue engineering.

According to embodiments of the present application, the recombinant collagen has a triple-helical conformation, thereby having biological activity and being able to be widely applied in various aspects such as beauty, medicine, and tissue engineering.

According to embodiments of the present application, the recombinant collagen monomers are homologous or heterologous.

According to embodiments of the present application, the recombinant collagen monomers are connected to each other through hydrogen bonds or electrostatic forces.

In a third aspect of the present application, the present application proposes a nucleic acid molecule. According to embodiments of the present application, the nucleic acid molecule encodes the recombinant collagen monomer according to the first aspect or the recombinant collagen according to the second aspect.

In a fourth aspect of the present application, the present application proposes a vector. According to embodiments of the present application, the vector includes the nucleic acid molecule according to the third aspect.

In a fifth aspect of the present application, the present application proposes a recombinant cell. According to embodiments of the present application, the recombinant cell includes the nucleic acid molecule according to the third aspect, the vector according to the fourth aspect, or expresses the recombinant collagen monomer according to the first aspect or the recombinant collagen according to the second aspect.

According to embodiments of the present application, the recombinant cell is a eukaryotic cell or a prokaryotic cell.

According to embodiments of the present application, the eukaryotic cell is Pichia pastoris, Saccharomyces cerevisiae, an animal cell, or a plant cell.

According to embodiments of the present application, the prokaryotic cell is Escherichia coli, Bacillus subtilis, or Bacillus licheniformis.

In a sixth aspect of the present application, the present application proposes a method for preparing a recombinant collagen. According to embodiments of the present application, the method includes: culturing the recombinant cell according to the fifth aspect under conditions suitable for protein expression, so as to obtain the recombinant collagen.

In a seventh aspect of the present application, the present application proposes the use of a reagent in the preparation of a medicament, wherein the medicament is for increasing the activity of a collagen or promoting the expression or secretion of a collagen, the reagent is for activating a collagen active fragment, and the collagen active fragment includes at least three GER sites and/or GEK sites and/or RGD sites and/or GEN sites.

In an eighth aspect of the present application, the present application proposes the use of a reagent in the preparation of a medicament, wherein the medicament is for inhibiting the activity of a collagen or inhibiting the expression or secretion of a collagen, the reagent is for inhibiting a collagen active fragment, and the collagen active fragment includes at least three GER sites and/or GEK sites and/or RGD sites and/or GEN sites.

According to embodiments of the present application, the use of the reagent in the preparation of the medicament may further include at least one of the following additional technical features.

According to embodiments of the present application, the collagen active fragment is derived from amino acids at positions 168 to 1196 of a collagen.

According to embodiments of the present application, the collagen active fragment includes three GER sites and/or GEK sites and/or RGD sites and/or GEN sites.

According to embodiments of the present application, the collagen active fragment is a peptide segment between amino acid position 1089 and amino acid position 1172 of a collagen.

According to embodiments of the present application, the collagen active fragment is a peptide segment between amino acid position 234 and amino acid position 296 of a collagen.

According to embodiments of the present application, the collagen active fragment is a peptide segment between amino acid position 273 and amino acid position 311 of a collagen.

According to embodiments of the present application, the collagen active fragment is a peptide segment between amino acid position 288 and amino acid position 452 of a collagen.

According to embodiments of the present application, the collagen active fragment is a peptide segment between amino acid position 300 and amino acid position 485 of a collagen.

According to embodiments of the present application, the collagen active fragment is a peptide segment between amino acid position 444 and amino acid position 503 of a collagen.

According to embodiments of the present application, the collagen active fragment is a peptide segment between amino acid position 483 and amino acid position 512 of a collagen.

According to embodiments of the present application, the collagen active fragment is a peptide segment between amino acid position 501 and amino acid position 599 of a collagen.

According to embodiments of the present application, the collagen active fragment is a peptide segment between amino acid position 507 and amino acid position 686 of a collagen.

According to embodiments of the present application, the collagen active fragment is a peptide segment between amino acid position 591 and amino acid position 731 of a collagen.

According to embodiments of the present application, the collagen active fragment is a peptide segment between amino acid position 678 and amino acid position 806 of a collagen.

According to embodiments of the present application, the collagen active fragment is a peptide segment between amino acid position 726 and amino acid position 830 of a collagen.

According to embodiments of the present application, the collagen active fragment is a peptide segment between amino acid position 795 and amino acid position 833 of a collagen.

According to embodiments of the present application, the collagen active fragment is a peptide segment between amino acid position 798 and amino acid position 839 of a collagen.

According to embodiments of the present application, the collagen active fragment is a peptide segment between amino acid position 852 and amino acid position 866 of a collagen.

According to embodiments of the present application, the collagen active fragment is a peptide segment between amino acid position 828 and amino acid position 932 of a collagen.

According to embodiments of the present application, the collagen active fragment is a peptide segment between amino acid position 861 and amino acid position 995 of a collagen.

According to embodiments of the present application, the collagen active fragment is a peptide segment between amino acid position 927 and amino acid position 1094 of a collagen.

According to embodiments of the present application, the collagen active fragment is a peptide segment between amino acid position 987 and amino acid position **1103** of a collagen.

**In** a ninth aspect of the present application, the present application proposes the use of the recombinant collagen monomer according to the first aspect or the recombinant collagen according to the second aspect in the preparation of a medicament, wherein the medicament is for beauty, wound repair, or joint lubrication.

In a tenth aspect of the present application, the present application proposes the use of the recombinant collagen monomer according to the first aspect or the recombinant collagen according to the second aspect in the preparation of a food.

In an eleventh aspect of the present application, the present application proposes the use of the recombinant collagen monomer according to the first aspect or the recombinant collagen according to the second aspect for beauty, wound repair, or joint lubrication.

Additional aspects and advantages of the present application will be given in part in the following description, and in part will become apparent from the following description, or be learned through practice of the present application.

### Brief Description of the Drawings

The above and/or additional aspects and advantages of the present application will become apparent and readily understood from the description of the embodiments in conjunction with the following drawings, in which:
Fig. 1 is a gel electrophoresis image of a recombinant collagen Y109 stock solution according to an embodiment of the present application;
Fig. 2 is a deconvoluted mass spectrum for molecular weight identification of the recombinant collagen Y109 according to an embodiment of the present application;
Fig. 3 is a circular dichroism (CD) spectrum curve of the recombinant collagen Y109 at 20°C according to an embodiment of the present application;
Fig. 4 is a scatter plot of circular dichroism (CD) ellipticity at 221 nm for the recombinant collagen Y109 at different temperatures according to an embodiment of the present application;
Fig. 5 is a protein gel electrophoresis image of the resistance of recombinant collagen Y109 to trypsin digestion (wherein WYM refers to a commercially available similar product) according to an embodiment of the present application;
Fig. 6 is a graph showing test results of adhesion of different fragments to NIH3T3 cells according to an embodiment of the present application;
Fig. 7 is a graph showing test results of adhesion of fragments with different repeat numbers to NIH3T3 cells according to an embodiment of the present application;
Fig. 8 is a graph showing results of the effect of the recombinant collagen Y109 within a certain concentration range on the adhesion activity of NIH/3T3 cells according to an embodiment of the present application.

### Detailed Description

Embodiments of the present application are described in detail below. The embodiments described below are exemplary and are only used to explain the present application, and should not be construed as limitations on the present application.

### Definitions and Explanations

Unless otherwise stated or clearly contradicted by the context, the articles "a", "an", and "the" used herein are intended to include "at least one" or "one or more". Therefore, these articles as used herein refer to one or more than one (i.e., at least one) of the grammatical objects of the article. For example, "a component" refers to one or more components, i.e., it is possible that more than one component is considered to be employed or used in the implementation of the described embodiments.

As used herein, the terms "including" or "including" are open-ended expressions, meaning that the contents specified in the present application are included, but other aspects are not excluded.

As used herein, the terms "optionally", "optional", or "option" generally mean that the subsequently described event or circumstance may but does not necessarily occur, and that the description includes instances where the event or circumstance occurs and instances where it does not.

The present application proposes a recombinant collagen monomer, a recombinant collagen and uses thereof, a nucleic acid molecule, a vector, a recombinant cell, and a method for preparing a recombinant collagen, each of which will be described in detail below.

### Recombinant Collagen Monomer

In one aspect of the present application, a recombinant collagen monomer is proposed. This recombinant collagen monomer includes at least one collagen active fragment, and the collagen active fragment includes at least three GER sites and/or GEK sites and/or RGD sites and/or GEN sites, which may be the same or different. In this context, the "collagen active fragment" described in the present application refers to a peptide segment derived from amino acids between position 168 and position 1196 of a collagen (SEQ ID NO: 1). Specifically, the GER sites, GEK sites, RGD sites, and GEN sites are also derived from peptide segments within this range, and multiple GER sites and/or GEK sites and/or RGD sites and/or GEN sites can be continuous or discontinuous.

It should be noted that the "collagen" described in the present application is natural human type III collagen, and its full-length sequence is the amino acid sequence shown in SEQ ID NO: 1. The phrase "amino acids at positions 168 to 1196" refers to the sequence from amino acid position 168 to amino acid position 1196 of the natural human type III collagen, which is the helical region of mature human type III collagen.

MMSFVQKGSWLLLALLHPTIILAQQEAVEGGCSHLGQSYADRDVWKPEPCQICVCDSGSVLCDDI
ICDDQELDCPNPEIPFGECCAVCPQPPTAPTRPPNGQGPQGPKGDPGPPGIPGRNGDPGIPGQPGSPGSPG
PPGICESCPTGPQNYSPQYDSYDVKSGVAVGGLAGYPGPAGPPGPPGPPGTSGHPGSPGSPGYQGPPGE
PGQAGPSGPPGPPGAIGPSGPAGKDGESGRPGRPGERGLPGPPGIKGPAGIPGFPGMKGHRGFDGRNGE
KGETGAPGLKGENGLPGENGAPGPMGPRGAPGERGRPGLPGAAGARGNDGARGSDGQPGPPGPPGT
AGFPGSPGAKGEVGPAGSPGSNGAPGQRGEPGPQGHAGAQGPPGPPGINGSPGGKGEMGPAGIPGAPG
LMGARGPPGPAGANGAPGLRGGAGEPGKNGAKGEPGPRGERGEAGIPGVPGAKGEDGKDGSPGEPG
ANGLPGAAGERGAPGFRGPAGPNGIPGEKGPAGERGAPGPAGPRGAAGEPGRDGVPGGPGMRGMPGS

PGGPGSDGKPGPPGSQGESGRPGPPGPSGPRGQPGVMGFPGPKGNDGAPGKNGERGGPGGPGPQGPP
GKNGETGPQGPPGPTGPGGDKGDTGPPGPQGLQGLPGTGGPPGENGKPGEPGPKGDAGAPGAPGGKG
DAGAPGERGPPGLAGAPGLRGGAGPPGPEGGKGAAGPPGPPGAAGTPGLQGMPGERGGLGSPGPKG
DKGEPGGPGADGVPGKDGPRGPTGPIGPPGPAGQPGDKGEGGAPGLPGIAGPRGSPGERGETGPPGPA
GFPGAPGQNGEPGGKGERGAPGEKGEGGPPGVAGPPGGSGPAGPPGPQGVKGERGSPGGPGAAGFPG
ARGLPGPPGSNGNPGPPGPSGSPGKDGPPGPAGNTGAPGSPGVSGPKGDAGQPGEKGSPGAQGPPGAP
GPLGIAGITGARGLAGPPGMPGPRGSPGPQGVKGESGKPGANGLSGERGPPGPQGLPGLAGTAGEPGR
DGNPGSDGLPGRDGSPGGKGDRGENGSPGAPGAPGHPGPPGPVGPAGKSGDRGESGPAGPAGAPGPA
GSRGAPGPQGPRGDKGETGERGAAGIKGHRGFPGNPGAPGSPGPAGQQGAIGSPGPAGPRGPVGPSGP
PGKDGTSGHPGPIGPPGPRGNRGERGSEGSPGHPGQPGPPGPPGAPGPCCGGVGAAAIAGIGGEKAGGF
APYYGDEPMDFKINTDEIMTSLKSVNGQIESLISPDGSRKNPARNCRDLKFCHPELKSGEYWVDPNQG
CKLDAIKVFCNMETGETCISANPLNVPRKHWWTDSSAEKKHVWFGESMDGGFQFSYGNPELPEDVLD
VQLAFLRLLSSRASQNITYHCKNSIAYMDQASGNVKKALKLMGSNEGEFKAEGNSKFTYTVLEDGCT
KHTGEWSKTVFEYRTRKAVRLPIVDIAPYDIGGPDQEFGVDVGPVCFL (SEQ ID NO: 1)

In a preferred embodiment of the present application, the collagen active fragment is a peptide segment between amino acid position 1089 and amino acid position 1172 of a collagen, corresponding to the sequence shown in SEQ ID NO: 2, which is the 019 peptide segment described in the examples of the present application. The adhesion activity of the collagen active fragment within this range is highest.

In a preferred embodiment of the present application, the collagen active fragment is a peptide segment between amino acid position 234 and amino acid position 296 of a collagen, corresponding to the sequence shown in SEQ ID NO: 7, which is the 001 peptide segment described in the examples of the present application.

In a preferred embodiment of the present application, the collagen active fragment is a peptide segment between amino acid position 273 and amino acid position 311 of a collagen, corresponding to the sequence shown in SEQ ID NO: 8, which is the 002 peptide segment described in the examples of the present application.

In a preferred embodiment of the present application, the collagen active fragment is a peptide segment between amino acid position 288 and amino acid position 452 of a collagen, corresponding to the sequence shown in SEQ ID NO: 9, which is the 003 peptide segment described in the examples of the present application.

In a preferred embodiment of the present application, the collagen active fragment is a peptide segment between amino acid position 300 and amino acid position 485 of a collagen, corresponding to the sequence shown in SEQ ID NO: 10, which is the 004 peptide segment described in the examples of the present application.

In a preferred embodiment of the present application, the collagen active fragment is a peptide segment between amino acid position 444 and amino acid position 503 of a collagen, corresponding to the sequence shown in SEQ ID NO: 11, which is the 005 peptide segment described in the examples of the present application.

In a preferred embodiment of the present application, the collagen active fragment is a peptide segment between amino acid position 483 and amino acid position 512 of a collagen, corresponding to the sequence shown in SEQ ID NO: 12, which is the 006 peptide segment described in the examples of the present application.

In a preferred embodiment of the present application, the collagen active fragment is a peptide segment between amino acid position 501 and amino acid position 599 of a collagen, corresponding to the sequence shown in SEQ ID NO: 13, which is the 007 peptide segment described in the examples of the present application.

In a preferred embodiment of the present application, the collagen active fragment is a peptide segment between amino acid position 507 and amino acid position 686 of a collagen, corresponding to the sequence shown in SEQ ID NO: 14, which is the 008 peptide segment described in the examples of the present application.

In a preferred embodiment of the present application, the collagen active fragment is a peptide segment between amino acid position 591 and amino acid position 731 of a collagen, corresponding to the sequence shown in SEQ ID NO: 15, which is the 009 peptide segment described in the examples of the present application.

In a preferred embodiment of the present application, the collagen active fragment is a peptide segment between amino acid position 678 and amino acid position 806 of a collagen, corresponding to the sequence shown in SEQ ID NO: 16, which is the 010 peptide segment described in the examples of the present application.

In a preferred embodiment of the present application, the collagen active fragment is a peptide segment between amino acid position 726 and amino acid position 830 of a collagen, corresponding to the sequence shown in SEQ ID NO: 17, which is the 011 peptide segment described in the examples of the present application.

In a preferred embodiment of the present application, the collagen active fragment is a peptide segment between amino acid position 795 and amino acid position 833 of a collagen, corresponding to the sequence shown in SEQ ID NO: 18, which is the 012 peptide segment described in the examples of the present application.

In a preferred embodiment of the present application, the collagen active fragment is a peptide segment between amino acid position 798 and amino acid position 839 of a collagen, corresponding to the sequence shown in SEQ ID NO: 19, which is the 013 peptide segment described in the examples of the present application.

In a preferred embodiment of the present application, the collagen active fragment is a peptide segment between amino acid position 852 and amino acid position 866 of a collagen, corresponding to the sequence shown in SEQ ID NO: 20, which is the 014 peptide segment described in the examples of the present application.

In a preferred embodiment of the present application, the collagen active fragment is a peptide segment between amino acid position 828 and amino acid position 932 of a collagen, corresponding to the sequence shown in SEQ ID NO: 21, which is the 015 peptide segment described in the examples of the present application.

In a preferred embodiment of the present application, the collagen active fragment is a peptide segment between amino acid position 861 and amino acid position 995 of a collagen, corresponding to the sequence shown in SEQ ID NO: 22, which is the 016 peptide segment described in the examples of the present application.

In a preferred embodiment of the present application, the collagen active fragment is a peptide segment between amino acid position 927 and amino acid position 1094 of a collagen, corresponding to the sequence shown in SEQ ID NO: 23, which is the 017 peptide segment described in the examples of the present application.

In a preferred embodiment of the present application, the collagen active fragment is a peptide segment between amino acid position 987 and amino acid position 1103 of a collagen, corresponding to the sequence shown in SEQ ID NO: 24, which is the 018 peptide segment described in the examples of the present application.

In a specific embodiment of the present application, the collagen active fragment has an amino acid sequence as shown in SEQ ID NO: 2, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, or SEQ ID NO: 24. 
GPRGDKGETGERGAAGIKGHRGFPGNPGAPGSPGPAGQQGAIGSPGPAGPRGPVGPSGPPGKDGT
SGHPGPIGPPGPRGNRGER (SEQ ID NO: 2)
 GRPGRPGERGLPGPPGIKGPAGIPGFPGMKGHRGFDGRNGEKGETGAPGLKGENGLPGENGAP (SEQ ID NO: 7)
GEKGETGAPGLKGENGLPGENGAPGPMGPRGAPGERGRP (SEQ ID NO: 8) 
GLPGENGAPGPMGPRGAPGERGRPGLPGAAGARGNDGARGSDGQPGPPGPPGTAGFPGSPGAKG
EVGPAGSPGSNGAPGQRGEPGPQGHAGAQGPPGPPGINGSPGGKGEMGPAGIPGAPGLMGARGPPGPA
GANGAPGLRGGAGEPGKNGAKGEPGPRGERGEA (SEQ ID NO: 9)
 
GPRGAPGERGRPGLPGAAGARGNDGARGSDGQPGPPGPPGTAGFPGSPGAKGEVGPAGSPGSNG
APGQRGEPGPQGHAGAQGPPGPPGINGSPGGKGEMGPAGIPGAPGLMGARGPPGPAGANGAPGLRGG
AGEPGKNGAKGEPGPRGERGEAGIPGVPGAKGEDGKDGSPGEPGANGLPGAAGER (SEQ ID NO: 10)
 GPRGERGEAGIPGVPGAKGEDGKDGSPGEPGANGLPGAAGERGAPGFRGPAGPNGIPGEK (SEQ ID NO: 11)
GERGAPGFRGPAGPNGIPGEKGPAGERGAP (SEQ ID NO: 12) 
GEKGPAGERGAPGPAGPRGAAGEPGRDGVPGGPGMRGMPGSPGGPGSDGKPGPPGSQGESGRP
GPPGPSGPRGQPGVMGFPGPKGNDGAPGKNGERGGP (SEQ ID NO: 13)
 
GERGAPGPAGPRGAAGEPGRDGVPGGPGMRGMPGSPGGPGSDGKPGPPGSQGESGRPGPPGPSG
PRGQPGVMGFPGPKGNDGAPGKNGERGGPGGPGPQGPPGKNGETGPQGPPGPTGPGGDKGDTGPPGP
QGLQGLPGTGGPPGENGKPGEPGPKGDAGAPGAPGGKGDAGAPGERGPP (SEQ ID NO: 14)
 
GKNGERGGPGGPGPQGPPGKNGETGPQGPPGPTGPGGDKGDTGPPGPQGLQGLPGTGGPPGENG
KPGEPGPKGDAGAPGAPGGKGDAGAPGERGPPGLAGAPGLRGGAGPPGPEGGKGAAGPPGPPGAAG
TPGLQGMPGER (SEQ ID NO: 15)
 
GAPGERGPPGLAGAPGLRGGAGPPGPEGGKGAAGPPGPPGAAGTPGLQGMPGERGGLGSPGPK
GDKGEPGGPGADGVPGKDGPRGPTGPIGPPGPAGQPGDKGEGGAPGLPGIAGPRGSPGERGETGPP
(SEQ ID NO: 16)
 
GMPGERGGLGSPGPKGDKGEPGGPGADGVPGKDGPRGPTGPIGPPGPAGQPGDKGEGGAPGLPG
IAGPRGSPGERGETGPPGPAGFPGAPGQNGEPGGKGERGAP (SEQ ID NO: 17)
 GSPGERGETGPPGPAGFPGAPGQNGEPGGKGERGAPGEK (SEQ ID NO: 18) GERGETGPPGPAGFPGAPGQNGEPGGKGERGAPGEKGEGGPP (SEQ ID NO: 19) GERGAPGEKGEGGPPGVAGPPGGSGPAGPPGPQGVKGERGSP (SEQ ID NO: 20)

GAPGEKGEGGPPGVAGPPGGSGPAGPPGPQGVKGERGSPGGPGAAGFPGARGLPGPPGSNGNPG
PPGPSGSPGKDGPPGPAGNTGAPGSPGVSGPKGDAGQPGEK (SEQ ID NO: 21)

GERGSPGGPGAAGFPGARGLPGPPGSNGNPGPPGPSGSPGKDGPPGPAGNTGAPGSPGVSGPKGD
AGQPGEKGSPGAQGPPGAPGPLGIAGITGARGLAGPPGMPGPRGSPGPQGVKGESGKPGANGLSGERG
PP (SEQ ID NO: 22)

GQPGEKGSPGAQGPPGAPGPLGIAGITGARGLAGPPGMPGPRGSPGPQGVKGESGKPGANGLSG
ERGPPGPQGLPGLAGTAGEPGRDGNPGSDGLPGRDGSPGGKGDRGENGSPGAPGAPGHPGPPGPVGPA
GKSGDRGESGPAGPAGAPGPAGSRGAPGPQGPRGDK (SEQ ID NO: 23)

GLSGERGPPGPQGLPGLAGTAGEPGRDGNPGSDGLPGRDGSPGGKGDRGENGSPGAPGAPGHPG

PPGPVGPAGKSGDRGESGPAGPAGAPGPAGSRGAPGPQGPRGDKGETGERGAA (SEQ ID NO: 24)

The recombinant collagen monomer of the present application has no restriction on the number of collagen active fragments, as long as the verification characteristics in the adhesion examples can be achieved. Therefore, in the present application, the recombinant collagen monomer can include 2 to 12 collagen active fragments, such as 2, 3, 4, 5, 6, 7, 8, 9, 10, 11, or 12 collagen active fragments; preferably, it includes 6 collagen active fragments. The multiple collagen active fragments included in the recombinant collagen monomer can be the same or different.

In a specific embodiment of the present application, a stabilization sequence can also be added to the C-terminus of at least one collagen active fragment in the recombinant collagen monomer to improve the stability of the collagen active monomer. The amino acid sequence of the stabilization sequence is shown in SEQ ID NO: 6.
GPPGPCCGGG (SEQ ID NO: 6)

In a specific embodiment of the present application, the recombinant collagen monomer has the amino acid sequence shown in SEQ ID NO: 3 or SEQ ID NO: 25. The amino acid sequence shown in SEQ ID NO: 3 is the amino acid sequence of a protein in which the 019 peptide segment is repeated in tandem 6 times (namely, the Y109 molecule), and the amino acid sequence shown in SEQ ID NO: 25 is the amino acid sequence of a protein in which the 012 peptide segment is repeated in tandem 12 times (namely, the Y70 molecule).

GPRGDKGETGERGAAGIKGHRGFPGNPGAPGSPGPAGQQGAIGSPGPAGPRGPVGPSGPPGKDGT
SGHPGPIGPPGPRGNRGERGPRGDKGETGERGAAGIKGHRGFPGNPGAPGSPGPAGQQGAIGSPGPAGP
RGPVGPSGPPGKDGTSGHPGPIGPPGPRGNRGERGPRGDKGETGERGAAGIKGHRGFPGNPGAPGSPG
PAGQQGAIGSPGPAGPRGPVGPSGPPGKDGTSGHPGPIGPPGPRGNRGERGPRGDKGETGERGAAGIKG
HRGFPGNPGAPGSPGPAGQQGAIGSPGPAGPRGPVGPSGPPGKDGTSGHPGPIGPPGPRGNRGERGPRG
DKGETGERGAAGIKGHRGFPGNPGAPGSPGPAGQQGAIGSPGPAGPRGPVGPSGPPGKDGTSGHPGPIG
PPGPRGNRGERGPRGDKGETGERGAAGIKGHRGFPGNPGAPGSPGPAGQQGAIGSPGPAGPRGPVGPS
GPPGKDGTSGHPGPIGPPGPRGNRGERGPPGPCCGGG (SEQ ID NO: 3)

GSPGERGETGPPGPAGFPGAPGQNGEPGGKGERGAPGEKGSPGERGETGPPGPAGFPGAPGQNGE
PGGKGERGAPGEKGSPGERGETGPPGPAGFPGAPGQNGEPGGKGERGAPGEKGSPGERGETGPPGPAG
FPGAPGQNGEPGGKGERGAPGEKGSPGERGETGPPGPAGFPGAPGQNGEPGGKGERGAPGEKGSPGE
RGETGPPGPAGFPGAPGQNGEPGGKGERGAPGEKGSPGERGETGPPGPAGFPGAPGQNGEPGGKGERG
APGEKGSPGERGETGPPGPAGFPGAPGQNGEPGGKGERGAPGEKGSPGERGETGPPGPAGFPGAPGQN
GEPGGKGERGAPGEKGSPGERGETGPPGPAGFPGAPGQNGEPGGKGERGAPGEKGSPGERGETGPPGP
AGFPGAPGQNGEPGGKGERGAPGEKGSPGERGETGPPGPAGFPGAPGQNGEPGGKGERGAPGEKGPP
GPCCGGG (SEQ ID NO: 25)

### Recombinant Collagen

In another aspect of the present application, a recombinant collagen is proposed. This recombinant collagen is composed of three of the above recombinant collagen monomers. These three recombinant collagen monomers can form a triple-helical structure, so that the resulting recombinant collagen has high cell adhesion activity, biological activity, and good hydrophilicity, and can be widely applied in various aspects such as beauty, medicine, and tissue engineering.

### Nucleic Acid Molecule, Vector, Recombinant Cell

In yet another aspect of the present application, a nucleic acid molecule, a vector, and a recombinant cell are proposed. The nucleic acid molecule is used to encode the above recombinant collagen monomer or recombinant collagen. The vector includes the nucleic acid molecule. The recombinant cell contains the nucleic acid molecule or the vector, or expresses the above recombinant collagen monomer or recombinant collagen.

In a specific embodiment of the present application, the nucleic acid molecule encodes the recombinant collagen. The nucleotide sequence of the nucleic acid molecule is shown in SEQ ID NO: 4.

GGTCCAAGAGGTGATAAGGGTGAAACTGGTGAAAGAGGTGCTGCTGGTATTAAGGGTCATAG
AGGTTTTCCAGGTAACCCAGGTGCTCCAGGTTCTCCAGGTCCAGCTGGTCAACAAGGTGCTATTGG
TTCTCCAGGTCCAGCTGGTCCAAGAGGTCCAGTTGGTCCATCTGGTCCACCAGGTAAGGATGGTAC
TTCTGGTCATCCAGGTCCAATTGGTCCACCAGGTCCAAGAGGTAACAGAGGTGAAAGAGGTCCAA
GAGGTGATAAGGGTGAAACTGGTGAAAGAGGTGCTGCTGGTATTAAGGGTCATAGAGGTTTTCCAG
GTAACCCAGGTGCTCCAGGTTCTCCAGGTCCAGCTGGTCAACAAGGTGCTATTGGTTCTCCAGGTC
CAGCTGGTCCAAGAGGTCCAGTTGGTCCATCTGGTCCACCAGGTAAGGATGGTACTTCTGGTCATC
CAGGTCCAATTGGTCCACCAGGTCCAAGAGGTAACAGAGGTGAAAGAGGTCCAAGAGGTGATAAG
GGTGAAACTGGTGAAAGAGGTGCTGCTGGTATTAAGGGTCATAGAGGTTTTCCAGGTAACCCAGGT
GCTCCAGGTTCTCCAGGTCCAGCTGGTCAACAAGGTGCTATTGGTTCTCCAGGTCCAGCTGGTCCA
AGAGGTCCAGTTGGTCCATCTGGTCCACCAGGTAAGGATGGTACTTCTGGTCATCCAGGTCCAATT
GGTCCACCAGGTCCAAGAGGTAACAGAGGTGAAAGAGGTCCAAGAGGTGATAAGGGTGAAACTG
GTGAAAGAGGTGCTGCTGGTATTAAGGGTCATAGAGGTTTTCCAGGTAACCCAGGTGCTCCAGGTT
CTCCAGGTCCAGCTGGTCAACAAGGTGCTATTGGTTCTCCAGGTCCAGCTGGTCCAAGAGGTCCAG
TTGGTCCATCTGGTCCACCAGGTAAGGATGGTACTTCTGGTCATCCAGGTCCAATTGGTCCACCAGG
TCCAAGAGGTAACAGAGGTGAAAGAGGTCCAAGAGGTGATAAGGGTGAAACTGGTGAAAGAGGT
GCTGCTGGTATTAAGGGTCATAGAGGTTTTCCAGGTAACCCAGGTGCTCCAGGTTCTCCAGGTCCA
GCTGGTCAACAAGGTGCTATTGGTTCTCCAGGTCCAGCTGGTCCAAGAGGTCCAGTTGGTCCATCT
GGTCCACCAGGTAAGGATGGTACTTCTGGTCATCCAGGTCCAATTGGTCCACCAGGTCCAAGAGGT
AACAGAGGTGAAAGAGGTCCAAGAGGTGATAAGGGTGAAACTGGTGAAAGAGGTGCTGCTGGTA
TTAAGGGTCATAGAGGTTTTCCAGGTAACCCAGGTGCTCCAGGTTCTCCAGGTCCAGCTGGTCAAC
AAGGTGCTATTGGTTCTCCAGGTCCAGCTGGTCCAAGAGGTCCAGTTGGTCCATCTGGTCCACCAG
GTAAGGATGGTACTTCTGGTCATCCAGGTCCAATTGGTCCACCAGGTCCAAGAGGTAACAGAGGTG
AAAGAGGTCCACCAGGTCCATGTTGTGGTGGTGGT (SEQ ID NO: 4)

In the above specific embodiments, "nucleic acid molecule" is a collective term for deoxyribonucleic acid (DNA) and ribonucleic acid (RNA), which is a biological macromolecule compound polymerized from many nucleotide monomers and is one of the most basic substances of life. Nucleotide sequence refers to the arrangement order of bases in DNA or RNA. Nucleic acid molecules include cDNA, and in some cases, the nucleic acid molecule can be modified for use in the vector of the present application, such as for codon optimization. In some cases, for the purpose of cloning into a vector, the sequence can be designed to include terminal restriction site sequences. Nucleic acid molecules can be obtained from various sources, such as through polymerase chain reaction (PCR) amplification of a nucleic acid encoding the molecule within or isolated from one or more given cells.

In the above specific embodiments, "vector" refers to a self-replicating DNA molecule that transfers DNA fragments (genes of interest) to recipient cells in recombinant DNA technology of genetic engineering. As the most common and simplest vector in genetic engineering, it must include three parts: a genetic marker gene, a replication region, and a gene of interest. In addition to commonly used Escherichia coli plasmid vectors, many other artificially constructed plasmid vectors suitable for microorganisms, yeast, plants, and the like have also been developed. Vectors include but are not limited to: single-stranded, double-stranded, or partially double-stranded nucleic acid molecules; nucleic acid molecules including one or more free ends or no free ends (for example, circular); nucleic acid molecules including DNA, RNA, or both; and other types of polynucleotides known in the art. One type of vector is a "plasmid," which refers to a circular double-stranded DNA loop into which additional DNA fragments can be inserted, for example, through standard molecular cloning techniques. Certain vectors are capable of autonomous replication in the host cells into which they are introduced (for example, bacterial vectors having a bacterial origin of replication and episomal mammalian vectors). Other vectors (for example, non-episomal mammalian vectors) are integrated into the genome of a host cell after being introduced into the host cell, thereby replicating along with the host genome. Furthermore, certain vectors are capable of directing the expression of a gene of interest. Such vectors are referred to herein as "expression vectors." A recombinant expression vector can include a form suitable for expression of a nucleic acid in a host cell, meaning that the recombinant expression vector includes one or more regulatory elements that can be selected based on the host cell used for expression and can be operably linked to the nucleic acid sequence to be expressed.

In the above specific embodiments, "host cell" refers to any cell type that is susceptible to transformation, transfection, transduction, or the like with a nucleic acid construct or an expression vector including the polynucleotide of the present application. "Host cell" covers any progeny of a parental cell that is not completely identical to the parental cell due to mutations occurring during the replication process. The host cell can be any cell useful in the production of the recombinant humanized collagen of the present application. To produce the recombinant collagen, the nucleic acid encoding the recombinant collagen can be isolated and inserted into one or more vectors for further cloning and/or expression in the host cell. Such nucleic acids can be easily isolated and sequenced using conventional techniques (for example, by using oligonucleotide probes capable of specifically binding to a gene encoding the recombinant collagen). The host cell refers to a cell into which an exogenous nucleic acid has been introduced, including progeny of such a cell. Host cells include transformants and transformed cells, including primary transformed cells and progeny derived therefrom, regardless of the number of passages. Progeny may not be completely identical in nucleic acid content to the parent cell but may contain mutations. Methods for introducing vectors into host cells are well known; for example, electroporation is used to introduce the vector into the host cell, and the method can also include transfection, microinjection techniques, gene gun technology, liposome-mediated methods, and the like. The host cell is a prokaryotic cell or a eukaryotic cell. The host cell is selected from any one of Pichia pastoris, Saccharomyces cerevisiae, Escherichia coli, and Bacillus subtilis. Preferably, the prokaryotic cell is Escherichia coli; preferably, the eukaryotic cell is Pichia pastoris.

### Method for Preparing Recombinant Collagen

In yet another aspect of the present application, a method for preparing a recombinant collagen is proposed. The method includes culturing the above recombinant cell under conditions suitable for protein expression to obtain the recombinant collagen.

In a specific embodiment of the present application, the method for preparing recombinant collagen refers to the use of recombinant DNA or recombinant RNA technology to obtain a recombinant vector linked with a gene fragment capable of being translated into a protein of interest, which is then transferred into a host cell capable of expressing the protein of interest to express a specific recombinant protein molecule, and the protein obtained. The culture conditions are well known to those skilled in the art. The present application does not place any restrictions on the mode of protein expression, which can be determined as needed; for example, the mode of protein expression is induced expression.

### Uses

In yet another aspect of the present application, the use of a reagent in the preparation of a medicament is proposed. The medicament can be used to increase or inhibit the activity of a collagen or the expression or secretion of a collagen. The reagent is used to activate or inhibit a collagen active fragment, and the collagen active fragment includes at least three GER sites and/or GEK sites and/or RGD sites and/or GEN sites.

In a specific embodiment of the present application, the amino acid sequences of the GER sites, GEK sites, RGD sites, and/or GEN sites in the collagen active fragment are derived from amino acids at positions 168 to 1196 of a collagen. The "collagen" is natural human type III collagen, and its full-length sequence is the amino acid sequence shown in SEQ ID NO: 1. The phrase "amino acids at positions 168 to 1196" refers to the sequence from amino acid position 168 to amino acid position 1196 of natural human type III collagen, which is the helical region of mature human type III collagen.

In a preferred embodiment of the present application, the collagen active fragment is a peptide segment between amino acid position 1089 and amino acid position 1172 of a collagen. The adhesion activity of the collagen active fragment within this range is highest.

In a preferred embodiment of the present application, the collagen active fragment is a peptide segment between amino acid position 234 and amino acid position 296 of a collagen.

In a preferred embodiment of the present application, the collagen active fragment is a peptide segment between amino acid position 273 and amino acid position 311 of a collagen.

In a preferred embodiment of the present application, the collagen active fragment is a peptide segment between amino acid position 288 and amino acid position 452 of a collagen.

In a preferred embodiment of the present application, the collagen active fragment is a peptide segment between amino acid position 300 and amino acid position 485 of a collagen.

In a preferred embodiment of the present application, the collagen active fragment is a peptide segment between amino acid position 444 and amino acid position 503 of a collagen.

In a preferred embodiment of the present application, the collagen active fragment is a peptide segment between amino acid position 483 and amino acid position 512 of a collagen.

In a preferred embodiment of the present application, the collagen active fragment is a peptide segment between amino acid position 501 and amino acid position 599 of a collagen.

In a preferred embodiment of the present application, the collagen active fragment is a peptide segment between amino acid position 507 and amino acid position 686 of a collagen.

In a preferred embodiment of the present application, the collagen active fragment is a peptide segment between amino acid position 591 and amino acid position 731 of a collagen.

In a preferred embodiment of the present application, the collagen active fragment is a peptide segment between amino acid position 678 and amino acid position 806 of a collagen.

In a preferred embodiment of the present application, the collagen active fragment is a peptide segment between amino acid position 726 and amino acid position 830 of a collagen.

In a preferred embodiment of the present application, the collagen active fragment is a peptide segment between amino acid position 795 and amino acid position 833 of a collagen.

In a preferred embodiment of the present application, the collagen active fragment is a peptide segment between amino acid position 798 and amino acid position 839 of a collagen.

In a preferred embodiment of the present application, the collagen active fragment is a peptide segment between amino acid position 852 and amino acid position 866 of a collagen.

In a preferred embodiment of the present application, the collagen active fragment is a peptide segment between amino acid position 828 and amino acid position 932 of a collagen.

In a preferred embodiment of the present application, the collagen active fragment is a peptide segment between amino acid position 861 and amino acid position 995 of a collagen.

In a preferred embodiment of the present application, the collagen active fragment is a peptide segment between amino acid position 927 and amino acid position 1094 of a collagen.

In a preferred embodiment of the present application, the collagen active fragment is a peptide segment between amino acid position 987 and amino acid position 1103 of a collagen.

In yet another aspect of the present application, the use of the above recombinant collagen monomer or recombinant collagen in the preparation of a medicament is proposed, and the medicament is for beauty, wound repair, or joint lubrication.

In yet another aspect of the present application, the use of the above recombinant collagen monomer or recombinant collagen in the preparation of a food is proposed.

In yet another aspect of the present application, the use of the above recombinant collagen monomer or recombinant collagen for beauty, wound repair, or joint lubrication is proposed.

Embodiments of the present application are described in detail below. The embodiments described below are exemplary and are only used to explain the present application, and should not be construed as limitations on the present application. Where specific techniques or conditions are not specified in the examples, they are performed according to the techniques or conditions described in the literature within the field or according to the product instructions. The reagents or instruments used, where manufacturers are not specified, are conventional products that can be obtained through commercial purchase.

### Embodiment 1: Selection of Protein Sequences

The full-length sequence of natural human type III collagen (Abcam, ab7535) is SEQ ID NO: 1. Researchers designed multiple truncated peptide segments based on the helical region of mature human type III collagen (AA 168-1196) according to the amino acid sequences of adhesion-related sites such as GER and GEK. These include the 001 peptide segment (SEQ ID NO: 7), 002 peptide segment (SEQ ID NO: 8), 003 peptide segment (SEQ ID NO: 9), 004 peptide segment (SEQ ID NO: 10), 005 peptide segment (SEQ ID NO: 11), 006 peptide segment (SEQ ID NO: 12), 007 peptide segment (SEQ ID NO: 13), 008 peptide segment (SEQ ID NO: 14), 009 peptide segment (SEQ ID NO: 15), 010 peptide segment (SEQ ID NO: 16), 011 peptide segment (SEQ ID NO: 17), 012 peptide segment (SEQ ID NO: 18), 013 peptide segment (SEQ ID NO: 19), 014 peptide segment (SEQ ID NO: 20), 015 peptide segment (SEQ ID NO: 21), 016 peptide segment (SEQ ID NO: 22), 017 peptide segment (SEQ ID NO: 23), 018 peptide segment (SEQ ID NO: 24), and 019 peptide segment (SEQ ID NO: 2). Recombinant proteins were expressed using Pichia pastoris. After purification, the peptide segment with the highest adhesion activity was screened through cell adhesion experiments, which is the 019 peptide segment at positions 1089AA-1172AA (84 amino acids) of the Col3A1 gene (SEQ ID NO: 2). Using these 84 amino acids as a repeat unit, it is repeated in tandem N times (N is an integer greater than or equal to 1) without linkers between the repeat sequences. Optionally, a C-terminal stabilization sequence, namely GPPGPCCGGG (SEQ ID NO: 6), can be added to the C-terminus. The present application has no restriction on the number of repeat sequences, as long as the verification characteristics in the adhesion examples can be achieved. Preferably, the number of repeat sequences is 6, which is the Y109 molecule, as shown in SEQ ID NO: 3.

### Embodiment 2: Construction of the Yeast Expression Strain

1. For the Pichia pastoris expression system, researchers performed codon optimization on the nucleotide sequence corresponding to the Y109 sequence (SEQ ID NO: 3). The optimized nucleotide sequence is SEQ ID NO: 4. BGI was commissioned to perform gene synthesis. The synthesized gene fragment was inserted into the pPICZ alpha A expression vector through XhoI and NotI restriction enzyme sites to obtain pPICZ alpha A-Y109. The complete sequence of the plasmid is SEQ ID NO: 5. 
AGATCTAACATCCAAAGACGAAAGGTTGAATGAAACCTTTTTGCCATCCGACATCCACAGGTC
CATTCTCACACATAAGTGCCAAACGCAACAGGAGGGGATACACTAGCAGCAGACCGTTGCAAACG
CAGGACCTCCACTCCTCTTCTCCTCAACACCCACTTTTGCCATCGAAAAACCAGCCCAGTTATTGG
GCTTGATTGGAGCTCGCTCATTCCAATTCCTTCTATTAGGCTACTAACACCATGACTTTATTAGCCTG
TCTATCCTGGCCCCCCTGGCGAGGTTCATGTTTGTTTATTTCCGAATGCAACAAGCTCCGCATTACA
CCCGAACATCACTCCAGATGAGGGCTTTCTGAGTGTGGGGTCAAATAGTTTCATGTTCCCCAAATG
GCCCAAAACTGACAGTTTAAACGCTGTCTTGGAACCTAATATGACAAAAGCGTGATCTCATCCAAG
ATGAACTAAGTTTGGTTCGTTGAAATGCTAACGGCCAGTTGGTCAAAAAGAAACTTCCAAAAGTCG
GCATACCGTTTGTCTTGTTTGGTATTGATTGACGAATGCTCAAAAATAATCTCATTAATGCTTAGCGC
AGTCTCTCTATCGCTTCTGAACCCCGGTGCACCTGTGCCGAAACGCAAATGGGGAAACACCCGCTT
TTTGGATGATTATGCATTGTCTCCACATTGTATGCTTCCAAGATTCTGGTGGGAATACTGCTGATAGC
CTAACGTTCATGATCAAAATTTAACTGTTCTAACCCCTACTTGACAGCAATATATAAACAGAAGGAA
GCTGCCCTGTCTTAAACCTTTTTTTTTATCATCATTATTAGCTTACTTTCATAATTGCGACTGGTTCCA
ATTGACAAGCTTTTGATTTTAACGACTTTTAACGACAACTTGAGAAGATCAAAAAACAACTAATTAT
TCGAAACGATGAGATTTCCTTCAATTTTTACTGCTGTTTTATTCGCAGCATCCTCCGCATTAGCTGCT
CCAGTCAACACTACAACAGAAGATGAAACGGCACAAATTCCGGCTGAAGCTGTCATCGGTTACTC
AGATTTAGAAGGGGATTTCGATGTTGCTGTTTTGCCATTTTCCAACAGCACAAATAACGGGTTATTG
TTTATAAATACTACTATTGCCAGCATTGCTGCTAAAGAAGAAGGGGTATCTCTCGAGAAGAGAGAA
GCTGAAGCTGGTCCAAGAGGTGATAAGGGTGAAACTGGTGAAAGAGGTGCTGCTGGTATTAAGGG
TCATAGAGGTTTTCCAGGTAACCCAGGTGCTCCAGGTTCTCCAGGTCCAGCTGGTCAACAAGGTGC
TATTGGTTCTCCAGGTCCAGCTGGTCCAAGAGGTCCAGTTGGTCCATCTGGTCCACCAGGTAAGGA
TGGTACTTCTGGTCATCCAGGTCCAATTGGTCCACCAGGTCCAAGAGGTAACAGAGGTGAAAGAG
GTCCAAGAGGTGATAAGGGTGAAACTGGTGAAAGAGGTGCTGCTGGTATTAAGGGTCATAGAGGT
TTTCCAGGTAACCCAGGTGCTCCAGGTTCTCCAGGTCCAGCTGGTCAACAAGGTGCTATTGGTTCT
CCAGGTCCAGCTGGTCCAAGAGGTCCAGTTGGTCCATCTGGTCCACCAGGTAAGGATGGTACTTCT
GGTCATCCAGGTCCAATTGGTCCACCAGGTCCAAGAGGTAACAGAGGTGAAAGAGGTCCAAGAGG
 
TGATAAGGGTGAAACTGGTGAAAGAGGTGCTGCTGGTATTAAGGGTCATAGAGGTTTTCCAGGTAA
CCCAGGTGCTCCAGGTTCTCCAGGTCCAGCTGGTCAACAAGGTGCTATTGGTTCTCCAGGTCCAGC
TGGTCCAAGAGGTCCAGTTGGTCCATCTGGTCCACCAGGTAAGGATGGTACTTCTGGTCATCCAGG
TCCAATTGGTCCACCAGGTCCAAGAGGTAACAGAGGTGAAAGAGGTCCAAGAGGTGATAAGGGTG
AAACTGGTGAAAGAGGTGCTGCTGGTATTAAGGGTCATAGAGGTTTTCCAGGTAACCCAGGTGCTC
CAGGTTCTCCAGGTCCAGCTGGTCAACAAGGTGCTATTGGTTCTCCAGGTCCAGCTGGTCCAAGAG
GTCCAGTTGGTCCATCTGGTCCACCAGGTAAGGATGGTACTTCTGGTCATCCAGGTCCAATTGGTCC
ACCAGGTCCAAGAGGTAACAGAGGTGAAAGAGGTCCAAGAGGTGATAAGGGTGAAACTGGTGAA
AGAGGTGCTGCTGGTATTAAGGGTCATAGAGGTTTTCCAGGTAACCCAGGTGCTCCAGGTTCTCCA
GGTCCAGCTGGTCAACAAGGTGCTATTGGTTCTCCAGGTCCAGCTGGTCCAAGAGGTCCAGTTGGT
CCATCTGGTCCACCAGGTAAGGATGGTACTTCTGGTCATCCAGGTCCAATTGGTCCACCAGGTCCA
AGAGGTAACAGAGGTGAAAGAGGTCCAAGAGGTGATAAGGGTGAAACTGGTGAAAGAGGTGCTG
CTGGTATTAAGGGTCATAGAGGTTTTCCAGGTAACCCAGGTGCTCCAGGTTCTCCAGGTCCAGCTG
GTCAACAAGGTGCTATTGGTTCTCCAGGTCCAGCTGGTCCAAGAGGTCCAGTTGGTCCATCTGGTC
CACCAGGTAAGGATGGTACTTCTGGTCATCCAGGTCCAATTGGTCCACCAGGTCCAAGAGGTAACA
GAGGTGAAAGAGGTCCACCAGGTCCATGTTGTGGTGGTGGTTAGGCGGCCGCCAGCTTTCTAGAA
CAAAAACTCATCTCAGAAGAGGATCTGAATAGCGCCGTCGACCATCATCATCATCATCATTGAGTTT
GTAGCCTTAGACATGACTGTTCCTCAGTTCAAGTTGGGCACTTACGAGAAGACCGGTCTTGCTAGA
TTCTAATCAAGAGGATGTCAGAATGCCATTTGCCTGAGAGATGCAGGCTTCATTTTTGATACTTTTTT
ATTTGTAACCTATATAGTATAGGATTTTTTTTGTCATTTTGTTTCTTCTCGTACGAGCTTGCTCCTGATC
AGCCTATCTCGCAGCTGATGAATATCTTGTGGTAGGGGTTTGGGAAAATCATTCGAGTTTGATGTTTT
TCTTGGTATTTCCCACTCCTCTTCAGAGTACAGAAGATTAAGTGAGACCTTCGTTTGTGCGGATCCC
CCACACACCATAGCTTCAAAATGTTTCTACTCCTTTTTTACTCTTCCAGATTTTCTCGGACTCCGCGC
ATCGCCGTACCACTTCAAAACACCCAAGCACAGCATACTAAATTTCCCCTCTTTCTTCCTCTAGGGT
GTCGTTAATTACCCGTACTAAAGGTTTGGAAAAGAAAAAAGAGACCGCCTCGTTTCTTTTTCTTCGT
CGAAAAAGGCAATAAAAATTTTTATCACGTTTCTTTTTCTTGAAATTTTTTTTTTTAGTTTTTTTCTCT
TTCAGTGACCTCCATTGATATTTAAGTTAATAAACGGTCTTCAATTTCTCAAGTTTCAGTTTCATTTTT
CTTGTTCTATTACAACTTTTTTTACTTCTTGTTCATTAGAAAGAAAGCATAGCAATCTAATCTAAGGG
GCGGTGTTGACAATTAATCATCGGCATAGTATATCGGCATAGTATAATACGACAAGGTGAGGAACTA
AACCATGGCCAAGTTGACCAGTGCCGTTCCGGTGCTCACCGCGCGCGACGTCGCCGGAGCGGTCG
AGTTCTGGACCGACCGGCTCGGGTTCTCCCGGGACTTCGTGGAGGACGACTTCGCCGGTGTGGTC
CGGGACGACGTGACCCTGTTCATCAGCGCGGTCCAGGACCAGGTGGTGCCGGACAACACCCTGGC
CTGGGTGTGGGTGCGCGGCCTGGACGAGCTGTACGCCGAGTGGTCGGAGGTCGTGTCCACGAACT
TCCGGGACGCCTCCGGGCCGGCCATGACCGAGATCGGCGAGCAGCCGTGGGGGCGGGAGTTCGCC
CTGCGCGACCCGGCCGGCAACTGCGTGCACTTCGTGGCCGAGGAGCAGGACTGACACGTCCGACG
 
GCGGCCCACGGGTCCCAGGCCTCGGAGATCCGTCCCCCTTTTCCTTTGTCGATATCATGTAATTAGTT
ATGTCACGCTTACATTCACGCCCTCCCCCCACATCCGCTCTAACCGAAAAGGAAGGAGTTAGACAA
CCTGAAGTCTAGGTCCCTATTTATTTTTTTATAGTTATGTTAGTATTAAGAACGTTATTTATATTTCAAA
TTTTTCTTTTTTTTCTGTACAGACGCGTGTACGCATGTAACATTATACTGAAAACCTTGCTTGAGAAG
GTTTTGGGACGCTCGAAGGCTTTAATTTGCAAGCTGGAGACCAACATGTGAGCAAAAGGCCAGCA
AAAGGCCAGGAACCGTAAAAAGGCCGCGTTGCTGGCGTTTTTCCATAGGCTCCGCCCCCCTGACG
AGCATCACAAAAATCGACGCTCAAGTCAGAGGTGGCGAAACCCGACAGGACTATAAAGATACCAG
GCGTTTCCCCCTGGAAGCTCCCTCGTGCGCTCTCCTGTTCCGACCCTGCCGCTTACCGGATACCTGT
CCGCCTTTCTCCCTTCGGGAAGCGTGGCGCTTTCTCAATGCTCACGCTGTAGGTATCTCAGTTCGGT
GTAGGTCGTTCGCTCCAAGCTGGGCTGTGTGCACGAACCCCCCGTTCAGCCCGACCGCTGCGCCTT
ATCCGGTAACTATCGTCTTGAGTCCAACCCGGTAAGACACGACTTATCGCCACTGGCAGCAGCCAC
TGGTAACAGGATTAGCAGAGCGAGGTATGTAGGCGGTGCTACAGAGTTCTTGAAGTGGTGGCCTAA
CTACGGCTACACTAGAAGGACAGTATTTGGTATCTGCGCTCTGCTGAAGCCAGTTACCTTCGGAAA
AAGAGTTGGTAGCTCTTGATCCGGCAAACAAACCACCGCTGGTAGCGGTGGTTTTTTTGTTTGCAA
GCAGCAGATTACGCGCAGAAAAAAAGGATCTCAAGAAGATCCTTTGATCTTTTCTACGGGGTCTGA
CGCTCAGTGGAACGAAAACTCACGTTAAGGGATTTTGGTCATGAGATC (SEQ ID NO: 5)
2. After linearizing the plasmid pPICZ alpha A-Y109 using DraI or SalI, it was introduced into Pichia pastoris KM71 competent cells by electroporation. Transformants were then screened on Zeocin-resistant plates.
3. Small-scale expression identification: 6-8 colonies were picked into 2 mL of BMGY medium and cultured overnight at 28°C and 220 rpm until the OD600 reached 2 to 6 (logarithmic phase). Centrifugation was performed at room temperature to collect the cell pellet, which was then transferred to BMMY medium. Methanol (1%) was supplemented daily. After 3 days of induced expression, SDS-PAGE was used to detect the expression of recombinant collagen in the medium. Positive clones with high secretion were screened for scale-up production.

The 19 short peptide molecules were identified using the same method as described above to screen for the short peptide molecule with the highest expression level. After the secretory expression of the 19 short peptide molecules, the fermentation supernatant was directly concentrated 5-fold and then subjected to SDS-PAGE detection. The results showed that the expression level of the 019 molecule was relatively high.

### Embodiment 3: Induced Expression of Recombinant Collagen

1. Glycerol seeds were taken from an ultra-low temperature freezer at -80°C and thawed at room temperature. They were inoculated into a primary seed medium (including 1% yeast extract, 2% tryptone, 2% glycerol, and 0.2% histidine) at a ratio of 0.1% to 5%, and cultured at 28 to 30°C and 200 to 250 rpm for 20 to 30 hours.
2. The seeds were inoculated into a production tank medium (complete BSM mineral salts basal medium) at a ratio of 1% to 10%. The growth temperature was controlled at 28 to 30°C, the pH was 4 to 6, and the dissolved oxygen (DO) was greater than or equal to 10%.
3. When the dissolved oxygen rose back to more than 80%, a feed medium (including but not limited to glucose, glycerol, and yeast powder) was added until the OD of the culture reached 200 to 350, after which feeding was stopped.
4. After feeding was stopped, the dissolved oxygen rose back to 80% again. After starvation for 0.5 to 1 hour, the temperature was lowered to 22 to 28°C, and an induction medium was added for induced expression at a rate of 2 to 15 ml/L*h.
5. After induction for 24 to 48 hours, the cultivation was ended. The fermentation broth was centrifuged at a centrifugal force of 10,000 to 12,000 g to collect the fermentation supernatant.

### Embodiment 4: Purification Process of Recombinant Collagen

1. The supernatant prepared by fermentation in Example 3 was inactivated at a high temperature of 60°C and then separated and purified using an MMC mixed-mode chromatography column. The chromatography column was equilibrated with at least 8 column volumes of an equilibration buffer (50 mM NaAc, pH 5.0). The fermentation supernatant was loaded so that the protein bound to the chromatography column. Impurities were washed away with a wash buffer (20 mM PBS, 1 M NaCl, pH 7.0), and elution was performed with an elution buffer (20 mM Tris, 2 M NaCl, pH 9.0) to collect the eluate.
2. The collected eluate from the chromatography was subjected to ultrafiltration and buffer exchange into a phosphate solution (20 mM PBS, pH 7.0), and the protein of interest was collected as the flow-through from an anion exchange chromatography column.
3. The flow-through from the anion exchange chromatography was concentrated by ultrafiltration to obtain a collagen stock solution.

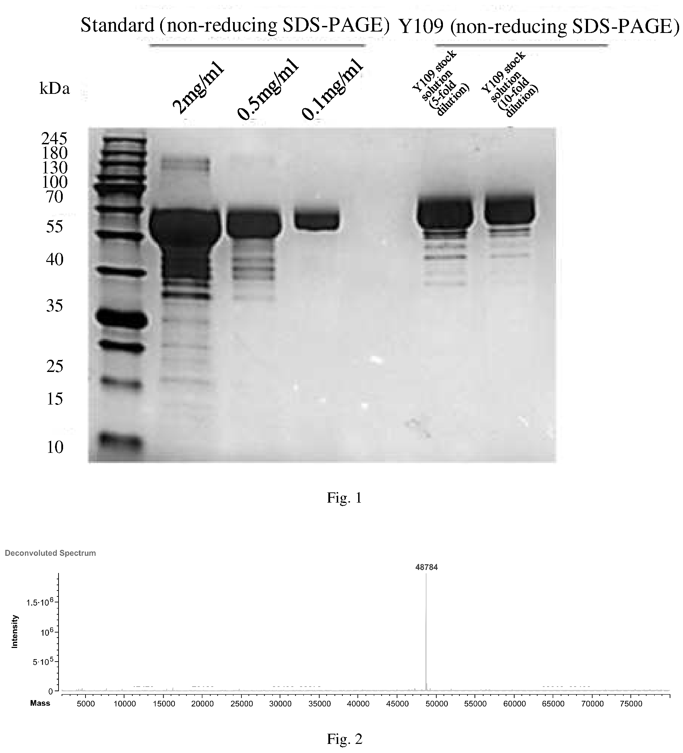

The Y109 collagen stock solution was prepared using the method described above and was subjected to gel electrophoresis detection. The results are shown in Fig. 1. The molecular weight is consistent with the theoretical molecular weight, and the purity after preliminary purification is approximately 85%.

### Embodiment 5: Mass Spectrometry Detection

1. Purified Y109 protein was prepared according to Embodiment 4.
2. The Y109 protein was diluted with ultrapure water to a final concentration of 1 mg/ml. A 200 µL aliquot was added to an autosampler vial.
3. Molecular weight detection of Y109 was performed using Liquid Chromatography-Mass Spectrometry (SEC-ESI-MS). The instrument used was a SCIEX high-resolution ZenoTOF 7600 mass spectrometry system, and the chromatographic column was an ACQUITY UPLC Protein BEH SEC. The injection volume was 1 µL. An isocratic elution mode was used (a 35% acetonitrile aqueous solution including 0.1% formic acid). Positive ion mode scanning was performed, with an ion source temperature set to 450°C, a spray voltage set to 5500 V, and a mass range (MS1) set to 300-2000 Da.
4. Data processing and analysis were performed using SCIEX Biologics Explorer software. Molecular weight identification of the protein was obtained based on the primary mass spectrum of the intact protein. Search parameters included: no fixed modifications; methionine oxidation, glutamine deamidation, and asparagine deamidation were set as variable modifications; and the protein library sequence was the Y109 sequence.
5. The deconvoluted mass spectrum for molecular weight identification is shown in Fig. 2. As can be seen from the figure, the deconvoluted molecular weight is 48,784 Da.
6. The detected molecular weight and the corresponding protein are shown in Table 1. It can be seen that the detected molecular weight is consistent with the theoretical molecular weight.

**Table 1**

| Protein | Post-translational Modification | Detected Molecular Weight (Da) | Theoretical Molecular Weight (Da) | Mass Deviation (ppm) |
|---|---|---|---|---|
| Y109 | None | 48783.8 | 48786.4 | -53.77 |

### Embodiment 6: Endotoxin Detection

1. Purified Y109 protein was prepared according to Embodiment 4.
2. Reagents were prepared, including a kinetic chromogenic Limulus Amebocyte Lysate (LAL) reagent (Zhanjiang A&C Biological Ltd., 0.005 to 50 EU/ml), water for bacterial endotoxin test (Xiamen LAL Reagent), and a working standard endotoxin series for photometry (Zhanjiang A&C Biological Ltd., 0.02 EU/ml, 0.2 EU/ml, 2 EU/ml).
3. The working standard endotoxin series for photometry was reconstituted into liquids of 0.02 EU/ml, 0.2 EU/ml, and 2 EU/ml. The samples were diluted 10-fold, 100-fold, and 2500-fold using water for bacterial endotoxin test.
4. The working standard endotoxin series for photometry, the diluted protein samples, the spiked protein samples (positive control), and the diluted buffer (negative control) were added to a microplate in sequence. Then, the kinetic chromogenic LAL reagent, which had been reconstituted with water for bacterial endotoxin test according to the labeled amount, was added to the microplate already containing the above samples.
5. The microplate was placed into a microplate reader. Changes in OD values within 1 hour were recorded at a wavelength of 405 nm and a temperature of 37°C. Corresponding software simultaneously displayed the curve and automatically fitted the standard curve to calculate the endotoxin content of the samples. After treatment, the endotoxin level of the Y109 protein could be controlled at 0.6 EU/mg.

### Embodiment 7: Detection of Triple-Helical Structure of Recombinant Collagen

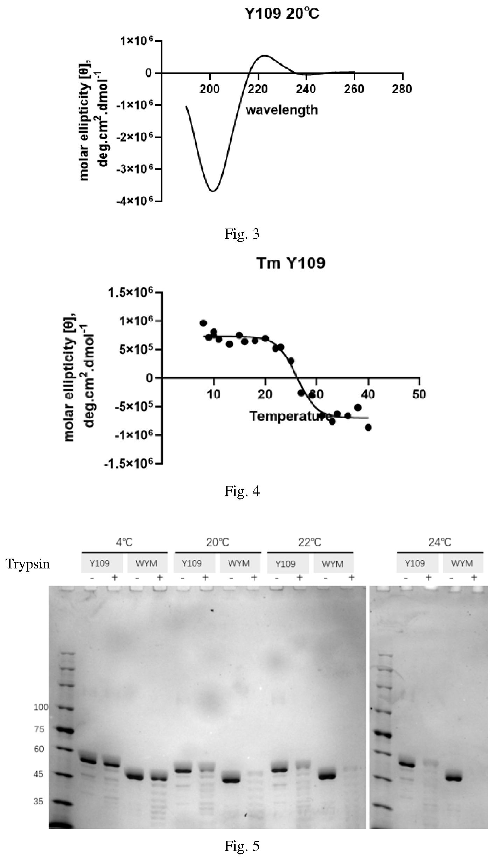

To confirm the tertiary structure of the recombinant collagen, circular dichroism was used to detect the collagen. First, the recombinant collagen was prepared into a 200 µg/mL protein solution using a 20 mM PB solution. The solution was left to stand overnight at 4°C before performing variable-temperature circular dichroism (CD) detection. The wavelength was set to 190-260 nm, the temperature was set from 4°C to 40°C, and the heating rate was 2°C/min. As shown in the CD spectrum in Fig. 3, at a low temperature (20°C), the curve has a negative peak near 195 nm and a positive peak near 221 nm. Furthermore, GraphPad was used to create a scatter plot of the ellipticity at 221 nm at different temperatures and to draw a fitting curve, as shown in Fig. 4. As the temperature rises, the positive peak near 221 nm disappears, indicating that the collagen has a triple-helical structure at low temperatures. As the temperature gradually increases, the collagen begins to unwind until it is completely unwound at 30°C. The Tm of the Y109 molecule was calculated to be approximately 26°C based on the curve.

### Embodiment 8: Detection of Triple-Helical Structure of Recombinant Collagen (Resistance to Trypsin Digestion)

Another method for detecting the triple-helical structure of collagen is the trypsin digestion resistance assay. Under specific conditions, trypsin at a certain concentration can degrade single-stranded collagen molecules, while collagen molecules that have formed a triple-helical structure can resist trypsin digestion; therefore, the triple-helical structure of collagen can be analyzed through the trypsin digestion resistance assay. First, the recombinant collagen was prepared into a 200 µg/mL protein solution using a 20 mM PB solution and left to stand overnight at 4°C. Trypsin was added at a ratio of 1 ng of trypsin per 1 µg of protein, and the protein molecules were subjected to digestion at different temperatures. After digestion for 1 hour, a non-reducing SDS-PAGE protein loading buffer was directly added, and SDS-PAGE was performed. As shown in Fig. 5, the Y109 molecule resists trypsin digestion at 4°C, and the Y109 molecule still possesses a certain degree of triple-helical structure at 24°C; therefore, the triple-helical structure of the Y109 molecule is stable at room temperature.

### Embodiment 9: Cell Activity Detection

### 1. Screening of fragments with pro-adhesion activity for NIH/3T3 cells

Candidate fragments (the 001 peptide segment to the 019 peptide segment in Embodiment 1) and controls (commercially available natural human collagen and commercially available recombinant humanized collagen) were diluted with PBS to a consistent final concentration (0.5 mg/ml). The negative control group (NC) was PBS buffer. The samples were added at 100 µL/well into a 96-well cell culture plate with an untreated culture surface, with three replicate wells per sample. After incubation at 4°C overnight, the cell culture plate was washed once with PBS. A 1% BSA solution was prepared, and 100 µL of 1% BSA was added to each well of the 96-well plate using a multi-channel pipette, followed by incubation at room temperature for 1 hour. After removing the BSA, 200 µL of PBS was added to each well to wash the wells once, and then the PBS was removed. The density of NIH/3T3 cells was adjusted to 1*10⁶ cells/mL. The cells were seeded into the 96-well plate using a multi-channel pipette at 100 µL per well (1*10⁵ cells/well) and incubated at 37°C for 1 hour. The medium was slowly removed, and the plate was washed four times with PBS. CCK8 reagent was prepared by adding 10 µL of CCK8 reagent per 100 µL of medium. After mixing evenly, 100 µL of the mixture was added per well into the 96-well plate. The plate was incubated in a 5% CO₂ cell incubator at 37°C for 1 hour and then taken out. The absorbance of the 96-well plate at 450 nm was read using a microplate reader, and the measurement results were recorded.

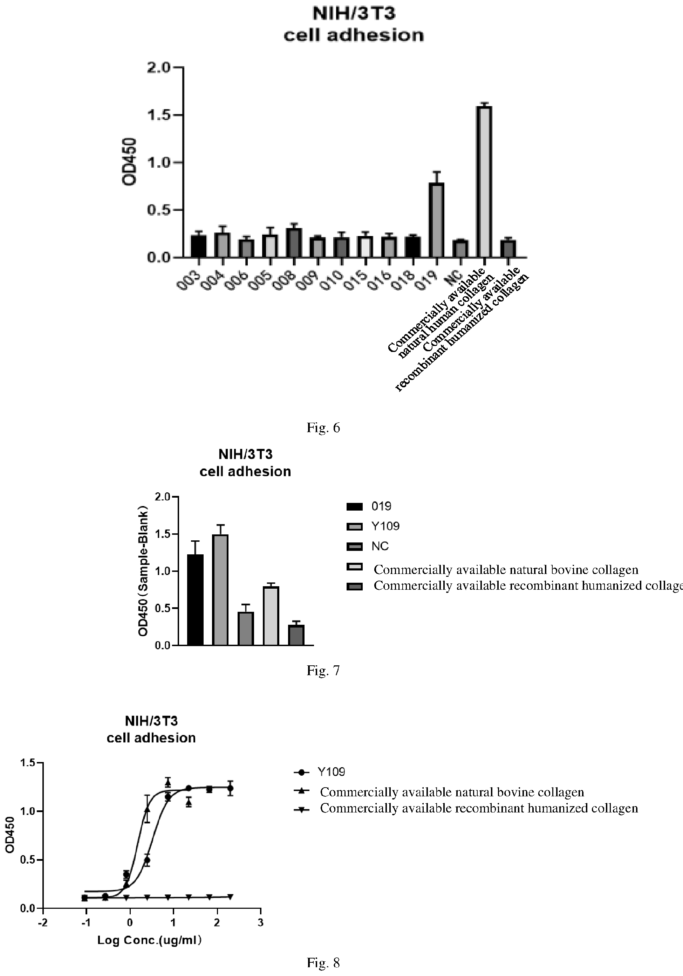

Natural type III collagen has activity in promoting the adhesion of NIH/3T3 cells. Cells that are not adhered or are weakly adhered to the bottom of the well can be removed by PBS washing; therefore, the effect of coating different molecules on cell adhesion activity can be reflected by detecting the number of remaining cells in different wells after PBS washing. After adding CCK8, the OD value detected at 450 nm is positively correlated with the cell number. A higher OD450 reflects that more cells adhere to the bottom of the well, and that the coated molecules have better activity in promoting NIH/3T3 cell adhesion. The experimental results are shown in Fig. 6. It can be seen that natural human type III collagen indeed has extremely strong activity in promoting NIH/3T3 cell adhesion, and among the candidate fragments, the 019 molecule shows significant activity in promoting NIH/3T3 cell adhesion.

### 2. Enhanced NIH/3T3 cell adhesion activity of Y109

The inventor(s) further tested the adhesion activity of molecules with different tandem repeat numbers based on the sequence of the 019 peptide segment and found that the adhesion activity correlates with the number of repeats. The cell adhesion activity is best when the number of repeat sequences is 6. Therefore, a repeat sequence number of 6, namely the Y109 molecule (SEQ ID NO: 3), was selected for subsequent research and testing. The nucleotide sequence encoding the Y109 molecule is SEQ ID NO: 4.

Candidate fragments (019 and Y109) and controls (commercially available natural bovine collagen and commercially available recombinant humanized collagen) were diluted with PBS to a consistent final concentration (0.2 mg/ml). The negative control group (NC) was PBS buffer. The samples were added at 100 µL/well into a 96-well cell culture plate with an untreated culture surface, with 3 wells per sample. After incubation at 4°C overnight, the cell culture plate was washed once with PBS. A 1% BSA solution was prepared, and 100 µL of 1% BSA was added to each well of the 96-well plate using a multi-channel pipette, followed by incubation at room temperature for 1 hour. After removing the BSA, 200 µL of PBS was added to each well to wash the wells once, and then the PBS was removed. The density of NIH/3T3 cells was adjusted to 1*10⁶ cells/mL. The cells were seeded into the 96-well plate using a multi-channel pipette at 100 µL/well (1*10⁵ cells/well) and incubated at 37°C for 1 hour. The medium was slowly removed, and the plate was washed four times with PBS (using a plate washer). CCK8 reagent was prepared by adding 10 µL of CCK8 reagent per 100 µL of medium. After mixing evenly, 100 µL of the mixture was added per well into the 96-well plate. The plate was incubated in a 5% CO₂ cell incubator at 37°C for 1 hour and then taken out. The absorbance of the 96-well plate at 450 nm was read using a microplate reader, and the measurement results were recorded. The experimental results are shown in Fig. 7. It can be seen that the Y109 molecule, obtained after repeating the candidate fragment 6 times, has stronger NIH/3T3 cell adhesion-promoting activity than the 019 molecule.

### 3. The NIH/3T3 cell adhesion-promoting activity of the Y109 molecule is concentration-dependent

Y109 and controls (commercially available natural bovine collagen and commercially available recombinant humanized collagen) were diluted with PBS to 200 µg/ml to prepare a total volume of 255 µL. After mixing evenly, 85 µL was taken and added to 170 µL of PBS, and so on, to perform serial dilutions 7 times for a total of 8 concentrations. After completing the dilutions, 50 µL/well was added to a 96-well cell culture plate with an untreated culture surface, with 3 replicate wells set for each concentration. After incubation at 4°C overnight, the cell culture plate was washed once with PBS. A 1% BSA solution was prepared, and 100 µL of 1% BSA was added to each well of the 96-well plate using a multi-channel pipette, followed by incubation at room temperature for 1 hour. After removing the BSA, 200 µL of PBS was added to each well to wash the wells once, and then the PBS was removed. The density of NIH/3T3 cells was adjusted to 1*10⁶ cells/mL. The cells were seeded into the 96-well plate using a multi-channel pipette at 100 µL/well (1*10⁵ cells/well) and incubated at 37°C for 1 hour. The medium was slowly removed, and the plate was washed four times with PBS (using a plate washer). CCK8 reagent was prepared by adding 10 µL of CCK8 reagent per 100 µL of medium. After mixing evenly, 100 µL of the mixture was added per well into the 96-well plate. The plate was incubated in a 5% CO₂ cell incubator at 37°C for 1 hour and then taken out. The absorbance of the 96-well plate at 450 nm was read using a microplate reader, and the measurement results were recorded. The experimental results are shown in Fig. 8. It can be seen that the NIH/3T3 cell adhesion-promoting activity of the Y109 molecule increases with concentration within a certain range.

In the description of this specification, reference to terms such as "one embodiment", "some embodiments", "an example", "a specific example", or "some examples" means that specific features, structures, materials, or characteristics described in connection with the embodiment or example are included in at least one embodiment or example of the present application. In this specification, the schematic expressions of the above terms are not necessarily directed to the same embodiment or example. Furthermore, the specific features, structures, materials, or characteristics described can be combined in a suitable manner in any one or more embodiments or examples. In addition, those skilled in the art can combine and group different embodiments or examples, as well as features of different embodiments or examples described in this specification, provided that they are not mutually contradictory.

Although embodiments of the present application have been shown and described above, it can be understood that the above embodiments are exemplary and should not be construed as limitations on the present application. Those of ordinary skill in the art can make changes, modifications, replacements, and variations to the above embodiments within the scope of the present application.

## Claims

1. A recombinant collagen monomer, comprising at least one collagen active fragment, wherein the collagen active fragment comprises at least three GER sites and/or GEK sites and/or RGD sites and/or GEN sites.

2. The recombinant collagen monomer of claim 1, wherein the collagen active fragment is derived from amino acids at positions 168 to 1196 of a collagen.

3. The recombinant collagen monomer of claim 1, wherein the collagen active fragment comprises three GER sites and/or GEK sites and/or RGD sites and/or GEN sites; and
the three GER sites and/or GEK sites and/or RGD sites and/or GEN sites are the same or different.

4. The recombinant collagen monomer of claim 1, wherein the collagen active fragment is a peptide segment between amino acid position 1089 and amino acid position 1172 of a collagen, and/or a peptide segment between amino acid position 234 and amino acid position 296 of the collagen, and/or a peptide segment between amino acid position 273 and amino acid position 311 of the collagen, and/or a peptide segment between amino acid position 288 and amino acid position 452 of the collagen, and/or a peptide segment between amino acid position 300 and amino acid position 485 of the collagen, and/or a peptide segment between amino acid position 444 and amino acid position 503 of the collagen, and/or a peptide segment between amino acid position 483 and amino acid position 512 of the collagen, and/or a peptide segment between amino acid position 501 and amino acid position 599 of the collagen, and/or a peptide segment between amino acid position 507 and amino acid position 686 of the collagen, and/or a peptide segment between amino acid position 591 and amino acid position 731 of the collagen, and/or a peptide segment between amino acid position 678 and amino acid position 806 of the collagen, and/or a peptide segment between amino acid position 726 and amino acid position 830 of the collagen, and/or a peptide segment between amino acid position 795 and amino acid position 833 of the collagen, and/or a peptide segment between amino acid position 798 and amino acid position 839 of the collagen, and/or a peptide segment between amino acid position 852 and amino acid position 866 of the collagen, and/or a peptide segment between amino acid position 828 and amino acid position 932 of the collagen, and/or a peptide segment between amino acid position 861 and amino acid position 995 of the collagen, and/or a peptide segment between amino acid position 927 and amino acid position 1094 of the collagen, and/or a peptide segment between amino acid position 987 and amino acid position 1103 of the collagen.

5. The recombinant collagen monomer of claim 1, wherein the collagen active fragment has an amino acid sequence as shown in SEQ ID NO: 2, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, or SEQ ID NO: 24.

6. The recombinant collagen monomer of claim 1, wherein the recombinant collagen monomer comprises 2 to 12 of the collagen active fragments;
the collagen active fragments are the same or different; and
the 2 to 12 collagen active fragments are connected directly or indirectly.

7. The recombinant collagen monomer of claim 6, wherein the recombinant collagen monomer comprises six of the collagen active fragments.

8. The recombinant collagen monomer of claim 1, wherein the recombinant collagen monomer further comprises a stabilization sequence, the stabilization sequence is connected to a C-terminus of the at least one collagen active fragment; and
an amino acid sequence of the stabilization sequence is SEQ ID NO: 6.

9. The recombinant collagen monomer of claim 1, wherein the recombinant collagen monomer has an amino acid sequence as shown in SEQ ID NO: 3 or SEQ ID NO: 25.

10. A recombinant collagen, comprising three of the recombinant collagen monomers of any one of claims 1 to 9.

11. The recombinant collagen of claim 10, wherein the recombinant collagen has a triple-helical conformation;
the recombinant collagen monomers are homologous or heterologous; and
the recombinant collagen monomers are connected to each other through hydrogen bonds or electrostatic forces.

12. A nucleic acid molecule, **characterized in that** it encodes the recombinant collagen monomer of any one of claims 1 to 9 or the recombinant collagen of any one of claims 10 to 11.

13. A vector, **characterized in that** it comprises the nucleic acid molecule of claim 12.

14. A recombinant cell, **characterized in that** it comprises the nucleic acid molecule of claim 12 or the vector of claim 13, or expresses the recombinant collagen monomer of any one of claims 1 to 9 or the recombinant collagen of any one of claims 10 to 11;
the recombinant cell is a eukaryotic cell or a prokaryotic cell;
the eukaryotic cell is Pichia pastoris, Saccharomyces cerevisiae, an animal cell, or a plant cell; and
the prokaryotic cell is Escherichia coli, Bacillus subtilis, or Bacillus licheniformis.

15. A method for preparing a recombinant collagen, comprising: culturing the recombinant cell of claim 14 under conditions suitable for protein expression, so as to obtain the recombinant collagen.

16. Use of a reagent in the preparation of a medicament, wherein the medicament is for increasing activity of a collagen or promoting expression or secretion of a collagen; the reagent is for activating a collagen active fragment; and the collagen active fragment comprises at least three GER sites and/or GEK sites and/or RGD sites and/or GEN sites.

17. Use of a reagent in the preparation of a medicament, wherein the medicament is for inhibiting activity of a collagen or inhibiting expression or secretion of a collagen; the reagent is for inhibiting a collagen active fragment; and the collagen active fragment comprises at least three GER sites and/or GEK sites and/or RGD sites and/or GEN sites.

18. The use of claim 16 or 17, wherein the collagen active fragment is derived from amino acids at positions 168 to 1196 of a collagen.

19. The use of claim 16 or 17, wherein the collagen active fragment comprises three GER sites and/or GEK sites and/or RGD sites and/or GEN sites.

20. The use of claim 16 or 17, wherein the collagen active fragment is a peptide segment between amino acid position 1089 and amino acid position 1172 of the collagen, and/or a peptide segment between amino acid position 234 and amino acid position 296 of the collagen, and/or a peptide segment between amino acid position 273 and amino acid position 311 of the collagen, and/or a peptide segment between amino acid position 288 and amino acid position 452 of the collagen, and/or a peptide segment between amino acid position 300 and amino acid position 485 of the collagen, and/or a peptide segment between amino acid position 444 and amino acid position 503 of the collagen, and/or a peptide segment between amino acid position 483 and amino acid position 512 of the collagen, and/or a peptide segment between amino acid position 501 and amino acid position 599 of the collagen, and/or a peptide segment between amino acid position 507 and amino acid position 686 of the collagen, and/or a peptide segment between amino acid position 591 and amino acid position 731 of the collagen, and/or a peptide segment between amino acid position 678 and amino acid position 806 of the collagen, and/or a peptide segment between amino acid position 726 and amino acid position 830 of the collagen, and/or a peptide segment between amino acid position 795 and amino acid position 833 of the collagen, and/or a peptide segment between amino acid position 798 and amino acid position 839 of the collagen, and/or a peptide segment between amino acid position 852 and amino acid position 866 of the collagen, and/or a peptide segment between amino acid position 828 and amino acid position 932 of the collagen, and/or a peptide segment between amino acid position 861 and amino acid position 995 of the collagen, and/or a peptide segment between amino acid position 927 and amino acid position 1094 of the collagen, and/or a peptide segment between amino acid position 987 and amino acid position 1103 of the collagen.

21. Use of the recombinant collagen monomer of any one of claims 1 to 9 or the recombinant collagen of any one of claims 10 to 11 in the preparation of a medicament, wherein the medicament is for beauty, wound repair, or joint lubrication.

22. Use of the recombinant collagen monomer of any one of claims 1 to 9 or the recombinant collagen of any one of claims 10 to 11 in the preparation of a food.

23. Use of the recombinant collagen monomer of any one of claims 1 to 9 or the recombinant collagen of any one of claims 10 to 11 for beauty, wound repair, or joint lubrication.
